# EUROPÄISCHE PATENTSCHRIFT

(11) **EP 1 041 955 B1**
(45) Veröffentlichungstag und Bekanntmachung des Hinweises auf die Patenterteilung: **03.09.2003**
(21) Anmeldenummer: 98965832.3
(22) Anmeldetag: 14.12.1998
(51) Int. Cl.: A61K 7/13

(54) **VERWENDUNG VON ÜBERGANGSMETALLKOMPLEXEN ZUM FÄRBEN VON KERATINHALTIGEN FASERN**
USE OF TRANSITION METAL COMPLEXES FOR DYEING KERATIN FIBERS
UTILISATION DE COMPLEXES DE METAUX DE TRANSITION POUR TEINDRE DES FIBRES KERATINIQUES

(30) Priorität: 23.12.1997 DE 19757510
(43) Veröffentlichungstag der Anmeldung: 11.10.2000
(73) Patentinhaber: Henkel Kommanditgesellschaft auf Aktien, 40589 Düsseldorf (DE)
(72) Erfinder: BLUM, Helmut, D-40595 Düsseldorf (DE); MAYER, Bernd, D-40597 Düsseldorf (DE)
(86) Internationale Anmeldenummer: EP9808192
(87) Internationale Veröffentlichungsnummer: WO99033435

(56) Entgegenhaltungen:
- EP-A- 0 150 676
- EP-A- 0 226 197
- EP-A- 0 630 964
- DE-A- 2 123 453
- DE-A- 2 327 987
- DE-A- 19 529 905
- FR-A- 2 735 976

## Beschreibung

Die vorliegende Erfindung betrifft die Verwendung von speziellen Übergangsmetallkomplexen als katalytisch wirksame Aktivatoren zum Färben keratinhaltiger Fasern, Färbemittel enthaltend diese Übergangsmetallkomplexe sowie ein Verfahren zum Färben von keratinhaltigen Fasern unter Verwendung dieser Übergangsmetallkomplexe.

Für das Färben von keratinhaltigen Fasern, z. B. Wolle, Pelzen und insbesondere menschlichen Haaren, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung.

Direktziehende Farbstoffe werden unter schonenden Bedingungen appliziert. Ihr Nachteil liegt jedoch darin, daß die Färbungen häufig nur über unzureichende Echtheitseigenschaften verfügen. Mit Oxidationsfarbstoffen lassen sich zwar intensive Färbungen mit guten Echtheitseigenschaften erzielen, die Entwicklung der Farbe geschieht jedoch in der Regel unter dem Einfluß von H₂O₂ oder H₂O₂-Addukten, was Schädigungen der Faser zur Folge haben kann.

Auch Oxidationsfärbemittel mit Luftsauerstoff als sehr mildem Oxidationsmittel wurden bereits vorgeschlagen; im allgemeinen laufen die Oxidationen mit Luftsauerstoff jedoch nicht vollständig ab. Insbesondere in Oxidationshaarfärbemitteln, die in der Regel in einem cremeartigen kosmetischen Träger appliziert werden, stößt der Luftsauerstoff auf erhebliche Diffusionsbarrieren. Weiterhin wurde, beispielsweise in der EP-B 1-0 548 620, vorgeschlagen, den Gehalt an Wasserstoffperoxid in den Oxidationsfärbemitteln auf 1 Gew.-% und weniger zu begrenzen, dem Mittel aber gleichzeitig zur Aktivierung spezielle Enzyme, Peroxidasen, zuzufügen. Alle diese Ansätze haben aber bisher nicht zu einem entscheidenden Durchbruch in Hinblick auf ein konkurenzfähiges Marktprodukt geführt.

Es besteht daher nach wie vor die Aufgabe, Oxidationsfärbemittel zu entwickeln, die aufgrund ihres geringeren Gehaltes an Oxidationsmitteln oder durch Oxidation mit Luftsauerstoff schonendere Färbungen ermöglichen, ohne das Färbeergebnis negativ zu beeinflussen. Weiterhin ist es in hohem Maße wünschenswert, den pH-Wert der Färbemittel auf einen Wert um den Neutralpunkt zu senken, um möglichen Schädigungen der Faser aufgrund der üblicherweise starken Alkalinität der Färbemittel vorzubeugen.

Es wurde nunmehr überraschenderweise gefunden, daß durch Verwendung spezieller Übergangsmetallkomplexe mit Liganden vom Salen-Typ in Oxidationsfärbemitteln
- die Menge des Oxidationsmittels stark reduziert werden kann und/oder
- die Ausfärbung alleine mit Luftsauerstoff als Oxidationsmittel erfolgen kann oder
- die Ausfärbung im neutralen pH-Bereich erfolgen kann.

Ein erster Gegenstand der Erfindung ist somit die Verwendung von Übergangsmetall(III)-Komplexen der Formel (I), 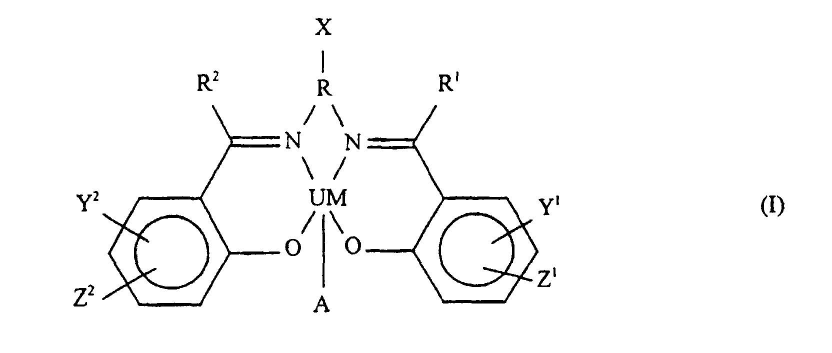 in der
- UM: für Mangan, Eisen, Kobalt, Ruthenium, Molybdän oder Vanadium steht,
- R: für einen Alkylen-, Alkenylen-, Phenylen- oder Cycloalkylenrest steht, welcher zusätzlich zum Substituenten X gegebenfalls alkyl- und/oder arylsubstituiert sein kann, mit insgesamt 1 bis 12 C-Atomen, wobei innerhalb R der kürzeste Abstand zwischen den mit UM komplexierenden N-Atomen 1 bis 5 C-Atome beträgt,
- X: für -H, -OR³, -NO₂, -F, -Cl, -Br oder -J steht,
- R¹, R² und R³: unabhängig voneinander für Wasserstoff oder einen Alkylrest mit 1 bis 4 C-Atomen stehen,
- Y¹ und Y²: unabhängig voneinander für Wasserstoff oder einen elektronenverschiebenden Substituenten stehen,
- Z¹ und Z²: unabhängig voneinander für Wasserstoff, -CO₂M, -SO₃M, -NO₂, -CH₃, -C₂H₅, -C₃H₇, -CH₂OH oder -CHO stehen,
- M: für Wasserstoff oder ein Alkalimetall wie Lithium, Natrium oder Kalium steht und
- A: für einen ladungsausgleichenden Anionliganden steht,
zum Färben keratinhaltiger Fasern.

Komplexe der Formel (I) sind bereits aus der WO-A1-97/07192 als Aktivatoren für Persauerstoffverbindungen in Oxidations-, Wasch-, Reinigungs- oder Desinfektionslösungen bekannt. Aufgrund der unterschiedlichen Vorgänge beim Bleichen und oxidativen Färben von Haaren konnte der Fachmann dieser Offenbarung aber keinerlei Hinweis auf die Eignung dieser Verbindung im Rahmen der sehr komplexen Vorgänge bei der oxidativen Entwicklung von Färbemitteln, insbesondere unter Aktivierung von Luftsauerstoff, entnehmen.

Bevorzugte Übergangsmetalle (UM in Formel I) sind Mangan, Eisen, Kobalt, Ruthenium und Vanadium; Komplexe mit Mangan als Übergangsmetall haben sich als erfindungsgemäß besonders geeignet erwiesen.

Zu den bevorzugten Verbindungen gemäß Formel (I) gehören solche, in denen R eine Methylengruppe, 1,2-Ethylengruppe, 1,3-Propylengruppe, in Position 2 hydroxy- oder nitrosubstituierte 1,3-Propylengruppe, 1,2-Cylcloalkylengruppe mit 4 bis 6 C-Atomen, insbesondere eine 1,2-Cyclohexylengruppe, oder eine o-Phenylengruppe ist. Die 1,2-Cyclohexylengruppe, insbesondere wenn sie keine Substituenten X außer Wasserstoff trägt, ist eine besonders bevorzugte Gruppe R.

Zu den elektronenverschiebenden Substitutenten Y¹ und Y² in Formel (I) gehören die Hydroxygruppe, Alkoxygruppen mit 1 bis 4 C-Atomen, Aryloxygruppen, die Nitrogruppe, Halogene wie Fluor, Chlor, Brom und Jod, die Aminogruppe, welche auch mono- oder dialkyliert oder -aryliert sein kann, lineare oder verzweigtkettige Alkylgruppen mit 1 bis 4 C-Atomen, Cycloalkylgruppen mit 3 bis 6 C-Atomen, lineare oder verzweigtkettige Alkenylgruppen mit 2 bis 5 C-Atomen, bevorzugt mit mindestens einer Doppelbindung in Konjugation zum Benzolring wie insbesondere die Vinyl- und die Allylgruppe, Arylgruppen, weiche ihrerseits die vorgenannten Substituenten tragen können, sowie die Gruppen -CHO, -COOM und -SO₃M. Bevorzugte Gruppen Y¹ und Y² sind die Hydroxygruppe, die Hydroxymethylgruppe, die Methoxygruppe, die Ethoxygruppe, die Phenyloxygruppe, die Nitrogruppe, Brom, die Vinylgruppe sowie die Gruppen -CHO, -COOH und -SO₃H. Vorzugsweise stehen die Substituenten Y¹ und Y² in 5-Stellung. Zu den bevorzugt verwendeten Verbindungen gemäß Formel (I) gehören solche, bei denen Y¹ und Y² identisch sind. Gemäß einer bevorzugten Ausführungsform stehen beide Substituenten Y¹ und Y² für Wasserstoff.

Zu den Alkylresten mit 1 bis 4 C-Atomen, insbesondere R¹, R² und R³, gehören insbesondere die Methyl-, Ethyl-, n-Propyl-, iso-Propyl-, n-Butyl-, sec-Butyl-, iso-Butylund tert-Butyl-Gruppe.

Bevorzugt stehen die Gruppen R¹ und R² für Wasserstoff.

Bevorzugte Gruppen Z¹ und Z² sind Wasserstoff.

Der ladungsausgleichende Anionligand A in den Verbindungen der Formel (I) kann einoder mehrwertig sein, wobei er im letzteren Fall entsprechend mehrere Übergangsmetall-Atome mit den genannten organischen Liganden neutralisieren kann. Vorzugsweise handelt es sich um ein Halogenid, insbesondere Chlorid, ein Hydroxid, Hexafluorophosphat, Perchlorat oder um das Anion einer Carbonsäure, wie Formiat, Acetat, Benzoat oder Citrat. Besonders bevorzugte Anionliganden A sind Halogenide, Carboxylat sowie Hexafluorophosphat.

Die erfindungsgemäß verwendeten Verbindungen gemäß Formel (I) können nach im Prinzip bekannten Verfahren durch die Reaktion von Salicylaldehyd oder entsprechenden Ketonen (wenn R¹ und/oder R² ungleich Wasserstoff), welche gegebenenfalls die oben definierten Substituenten Y¹, Y², Z¹ und/oder Z² tragen, mit Diaminen H₂N-R-NH₂ und der Umsetzung des so erhältlichen Salen-Liganden mit Übergangsmetallsalzen hergestellt werden, wie dies zum Beispiel in der europäischen Patentanmeldung EP 630 694 oder von B.B. De, B.B. Lohraj, S. Sivaram und P.K. Dhal in Macromolecules 27 (1994), 1291-1296 beschrieben worden ist. Auf diese Literaturstellen wird ausdrücklich Bezug genommen.

Beispiel für erfindungsgemäß verwendbare Komplexe der Formel (I) sind:
- {N,N'-Bis[(2-hydroxyphenyl)methylen]-1,2-diaminoethan}mangan(III)-chlorid
- {N,N'-Bis[(2-hydroxyphenyl)methylen]-1,2-diaminocyclohexan}mangan(III)-chlorid
- {N,N'-Bis[(2-hydroxyphenyl)methylen]-1,2-diaminocyclohexan}mangan(III)-acetat
- {N,N'-Bis[(2-hydroxy-5-vinylphenyl)methylen]-1,2-diaminocyclohexan}mangan(III)-chlorid
- {N,N'-Bis[(2-hydroxyphenyl)methylen]-1,3-diamino-2-propanol}mangan(III)-chlorid
- {N,N'-Bis[(2-hydroxyphenyl)methylen]-1,3-diamino-2-propanol}mangan(III)-acetat
- {N,N'-Bis[(2-hydroxy-5-vinyl-phenyl)methylen]-1,3-diamino-2-propanol}mangan(III)-chlorid
- {N,N'-Bis[(2-hydroxy-5-vinyl-phenyl)methylen]-1,3-diamino-2-propanol}mangan(III)-acetat
- {N,N'-Bis[(2-hydroxy-5-sulfonatophenyl)methylen]-1,2-diaminocyclohexan}mangan(III)-chlorid
- {N,N'-Bis[(2-hydroxy-5-nitrophenyl)methylen]-1,2-diaminocyclohexan}mangan(III)-acetat

Unter diesen Komplexen erfindungsgemäß bevorzugt sind {N,N'-Bis[(2-hydroxyphenyl)methylen]-1,2-diaminoethan}mangan(III)-chlorid, {N,N'-Bis[(2-hydroxyphenyl)methylen]-1,2-diaminocyclohexan}mangan(III)-Chlorid, {N,N'-Bis[(2-hydroxyphenyl)methylen]-1,2-diaminocyclohexan}mangan(III)-acetat, {N,N'-Bis[(2-hydroxy-5-vinylphenyl)methylen]-1.2-diaminocyclohexan}-mangan(III)-chlorid, {N,N'-Bis[(2-hydroxyphenyl)methylen]-1,3-diamino-2-propanol}mangan(III)-acetat, {N,N'-Bis[(2-hydroxy-5-sulfonatophenyl)methylen]-1,2-diaminocyclohexan}-mangan(III)-chlorid und {N,N'-Bis[(2-hydroxy-5-nitrophenyl)methylen]-1,2-diaminocyclohexan}-mangan(III)-acetat.

Als ganz besonders gut geeignet haben sich die Komplexe {N,N'-Bis[(2-hydroxyphenyl)methylen]-1,2-diaminocyclohexan}mangan(III)-Chlorid, {N,N'-Bis[(2-hydroxyphenyl)methylen]-1,2-diaminocyclohexan}mangan(III)-acetat, {N,N'-Bis[(2-hydroxy-5-vinylphenyl)methylen]-1,2-diaminocyclohexan}-mangan(III)-chlorid und {N,N'-Bis[(2-hydroxy-5-nitrophenyl)methylen]-1,2-diaminocyclohexan}-mangan(III)-acetat erwiesen.

Ein zweiter Gegenstand der Erfindung sind Mittel zum Färben keratinischer Fasern, die mindestens ein Oxidationsfarbstoffvorprodukt sowie 0,001 bis 1,0 Gew.-%, bevorzugt 0,1 bis 1,0 Gew.-%, mindestens einer Verbindung gemäß Formel (I) enthalten.

Gemäß einer ersten bevorzugten Ausführungsform enthalten die erfindungsgemäßen Mittel übliche Oxidationsfarbstoffvorprodukte vom Typ der Entwickler und gewünschtenfalls vom Typ der Kuppler.

Entwicklersubstanzen sind üblicherweise aromatische oder heterocyclische Ringsysteme, die durch zwei in ortho- oder para-Stellung zueinander stehende reaktive Gruppen, i.a. Hydroxy- oder Aminogruppen, gekennzeichnet sind. Solche Verbindungen sind beispielsweise primäre aromatische Amine mit einer weiteren, in para- oder ortho-Position befindlichen freien oder substituierten Hydroxy- oder Aminogruppe, ferner Diaminopyridinderivate, heterocyclische Hydrazonderivate oder 4-Aminopyrazolonderivate.

Erfindungsgemäß bevorzugte Entwicklerkomponenten sind p-Phenylendiamin, p-Toluylendiamin, p-Aminophenol, 2-(2,5-Diaminophenyl)-ethanol, N,N-Bis-(2-hydroxy-ethyl)-p-phenylendiamin, 2-(2,5-Diaminophenoxy)-ethanol, 1-Phenyl-3-carboxyamido-4-aminopyrazolon-5, 4-Amino-3-methylphenol, 2-Hydroxymethyl-4-aminophenol, 2-Aminomethyl-4-aminophenol, 2-Hydroxyethylaminomethyl-4-amino-phenol, 2,4,5,6-Tetraamino-pyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 4,4'-Di-aminodiphenylamin sowie deren physiologisch verträgliche Salze.

Kupplersubstanzen sind häufig aromatische oder heterocyclische Ringsysteme, die zwei reaktive Gruppen in meta-Stellung aufweisen. Beispiele für solche Substanzen sind m-Phenylendiaminderivate, m-Aminophenole, Naphthole, Resorcinderivate und Pyrazolone.

Erfindungsgemäß bevorzugte Kupplerkomponenten sind 1-Naphthol, Pyrogallol, 1,5-, 2,7- und 1,7-Dihydroxynaphthalin, o-Aminophenol, 5-Amino-2-methylphenol, m-Aminophenol, Resorcin, Resorcinmonomethylether, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 5-Amino-2-methyl-4-chlorphenol, 2-Methylresorcin, 5-Methylresorcin, 2,5-Diniethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 2,6-Bis-(2-hydroxyethylamino)-toluol, 2,4-Diaminophenoxyethanol, 2-Amino-4-hydroxyethylamino-anisol sowie deren physiologisch verträgliche Salze.

Besonders bevorzugte Kuppler-Komponenten sind 2,4-Diaminophenoxyethanol, Resorcin, 2-Methylresorcin, 2,5-Dimethylresorcin, 4-Chlorresorcin, 5-Amino-2-methylphenol, 5-Amino-2-methyl-4-chlorphenol, m-Aminophenol, 1-Naphthol, 2,7-Dihydroxynaphthalin und deren physiologisch verträgliche Salze.

Üblicherweise werden Entwicklerkomponenten und Kupplerkomponenten in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0,5 bis 1 : 2 im Färbemittel enthalten sein können. Die Gesamtmenge an Oxidationsfarbstoffvorprodukten liegt in der Regel bei höchstens 20 Gew.-%, bezogen auf das gesamte Mittel.

Gemäß einer zweiten Ausführungsform enthalten die erfindungsgemäßen Färbemittel als alleinige Oxidationsfarbstofrvorprodukte oder in Kombination mit den oben genannten Kuppler- oder Entwicklerkomponenten ein Indol- oder Indolin-Derivat mit mindestens einem Hydroxysubstituenten.

Bevorzugte Indol-Derivate sind 6-Hydroxyindol, N-Methyl-6-hydroxyindol, N-Ethyl-6-hydroxyindol, N-Propyl-6-hydroxyindol, N-Butyl-6-hydroxyindol, 4-Hydroxyindol, N-Methyl-4-hydroxyindol, N-Ethyl-4-hydroxyindol, N-Propyl-4-hydroxyindol, N-Butyl-4-hydroxyindol, 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol.

Bevorzugte Indolin-Derivate sind die Verbindungen der Formel (II) 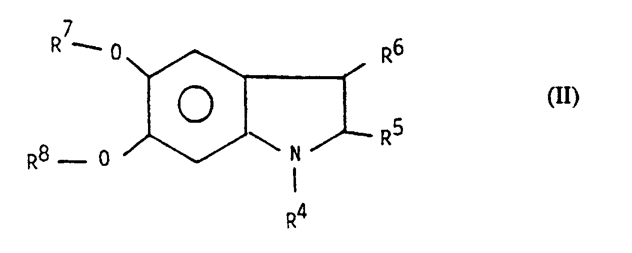 in der unabhängig voneinander
R⁴ steht für Wasserstoff oder eine C1-C4-Alkylgruppe,
R⁵ steht für Wasserstoff oder eine -COOH-Gruppe, wobei die -COOH-Gruppe auch als Salz mit einem physiologisch verträglichen Kation vorliegen kann,
R⁶ steht für Wasserstoff oder eine C1-C4-Alkylgruppe,
R⁷ steht für Wasserstoff, eine C1-C4-Alkylgruppe oder eine Gruppe -CO-R⁹, in der
R⁹ steht für eine C 1-C4-Alkylgruppe, und
R⁸ steht für eine der unter R⁷ genannten Gruppen,
oder ein physiologisch verträgliches Salz dieser Verbindungen mit einer organischen oder anorganischen Säure.

Erfindungsgemäß besonders bevorzugte Indolin-Derivate sind 5,6-Dihydroxyindolin, N-Methyl-5,6-dihydroxyindolin, N-Ethyl-5,6-dihydroxyindolin, N-Propyl-5,6-dihydroxyindolin und N-Butyl-5,6-dihydroxyindolin; besonders bevorzugt ist das 5,6-Dihydroxyindolin.

Neben den Oxidationsfarbstoffvorprodukten vom Kuppler- oder Entwicklertyp und/oder den Indol- oder Indolin-Derivaten können die erfindungsgemäßen Mittel weiterhin, insbesondere zur Nuancierung, direktziehende Farbstoffe und/oder natürliche Farbstoffe enthalten.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, ⁻HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol und 4-N-Ethyl-1,4-bis(2'-hydroxyethylamino)-2-nitrobenzol-hydrochlorid.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Bevorzugte, in der Natur vorkommende Farbstoffe sind beispielsweise Henna rot, Henna neutral, Henna schwarz, Kamillenblüte, Sandelholz, schwarzen Tee, Faulbaumrinde. Salbei, Blauholz, Krappwurzel. Catechu, Sedre und Alkannawurzel.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln. bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die in den erfindungsgemäßen Mitteln enthaltenen Farbstoffe oder Farbstoffvorprodukte mit Aminogruppen können sowohl als freie Basen als auch in Form ihrer physiologisch verträglichen Salze mit anorganischen oder organischen Säuren, z. B. der Hydrochloride, der Sulfate und Hydrobromide, eingesetzt werden.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, (Seiten 264-267; Oxidationsfarbstoffvorprodukte), erschienen als Band 7 der Reihe "Dermatology" (Hrg.: Ch.. Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Die erfindungsgemäßen Mittel eignen sich inbesondere zum Färben keratinischer Fasern.

Unter keratinischen Fasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten, insbesondere in der Farbphotographie, nichts entgegen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z. B. Cremes, Emulsionen, Gele oder auch tensidhaltige schäumende Lösungen, z. B. Shampoos, Schaumaerosole oder andere Zubereitungen, die für die Anwendung auf dem Haar geeignet sind.
Die erfindungsgemäßen Färbemittel können weiterhin alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Färbemittel alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-. Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(-CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylen glykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren. wie Ölsäure, Stearinsäure, Isostearinsäure und Palmitinsäure. Nichtionische Tenside enthalten als hydrophile Gruppe z. B. eine Polyolgruppe, eine Polyalkylenglykolethergruppe oder eine Kombination aus Polyol- und Polyglykol-ethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C12-C22-Fettsäuremono- und -diester von Anlagerungsprodukten von 1 bis 30 Mol Ethylenoxid an Glycerin,
- C8-C22-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethylammonium-glycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethyl-hydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C8-C18-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C12-18-Acylsarcosin.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldi-methylammoniumchloride und Trialkylmethylammoniumchloride, z. B. Cetyltrimethyl-ammoniumchlorid, Stearyltrimethylammoniumchlorid, Distearyldimethylammonium-chlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyl-dialkoyloxyalkyl-ammonium-methosulfate sowie die unter dem Warenzeichen Dehyquart® vertriebenen Produkte.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Je nach Art des Mittels und des Tensidtyps sind die Tenside in den erfindungsgemäßen Mitteln üblicherweise in Mengen von insgesamt 0,5 bis 30 Gew.-%, bezogen auf das gesamte Mittel, enthalten.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten. Bevorzugte kationische Polymere sind beispielsweise
- quatemisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quatemierte Cellulose-Derivate.
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche kationischen Polymere.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan. sowie deren alkoxylierte und quaternierte Analoga. Beispiele für solche Silikone sind die von Dow Corning unter den Bezeichnungen DC 190, DC 200, DC 344, DC 345 und DC 1401 vertriebenen Produkte sowie die Handelsprodukte Q2-7224 (Hersteller; Dow Corning; ein stabilisiertes Trimethylsilylamodimethicon), Dow Corning® 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Ebenfalls einsetzbar als konditionierende Wirkstoffe sind Paraffinöle sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide. beispielsweise Sojalecithin, Ei-Lecithin und Kephaline.

Schließlich enthalten die erfmdungsgemäßen Färbemittel bevorzugt noch einen Fettstoff.

Bevorzugte Fettstoffe sind lineare und verzweigte, gesättigte und ungesättigte Fettalkohole oder natürliche Fettalkoholgemisch mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Palmitylalkohol, Laurylalkohol, Myristylalkohol, Arachidylalkohol, Caprylalkohol. Caprinalkohol, Linoleylalkohol, Linolenylalkohol und Behenylalkohol, sowie deren Guerbetalkohole sowie Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0,01 bis 15 Gew.-%, bevorzugt von 0,1 bis 10 Gew.-% und besonders bevorzugt von 0,3 bis 6 Gew.-%, bezogen auf die gesamte Zubereitung. eingesetzt.

Ebenfalls als Fettstoffe eingesetzt werden können Monoester der Fettsäuren mit Alkoholen mit 6 bis 24 C-Atomen sowie Triglyceride natürlichen Ursprungs.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methylmethacrylat/tert.Butylaminoethylroethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/N-tert.-Butylacrylamid-Terpolymere,
- symmetrische und unsymmetrische, lineare und verzweigte Dialkylether mit insgesamt zwischen 12 bis 36 C-Atomen. insbesondere 12 bis 24 C-Atomen, beispielsweise Di-n-octylether. Di-n-decylether, Di-n-nonylether, Di-n-undecylether und Di-n-dodecylether, n-Hexyl-n-octylether, n-Octyl-n-decylether, n-Decyl-nundecylether, n-Undecyl-n-dodecylether und n-Hexyl-n-undecylether sowie Di-tertbutylether, Di-iso-pentylether, Di-3-ethyldecylether, tert-Butyl-n-octylether, iso-Pentyl-n-octylether und 2-Methyl-pentyl-n-octylether,
- Antischuppenwirkstoffe wie Piroctone Olamine, Zink Omadine und Climbazol,
- tierische und pflanzliche Proteinhydrolysate, insbesondere Elastin-, Kollagen-, Keratin-, Milcheiweiß-, Sojaprotein-, Mandelprotein- und Weizenproteinhydrolysate, sowie deren Fettsäurekondensationsprodukte und quaternierte Derivate,
- Vitamine und Vitaminvorstufen wie Panthenol, dessen Derivate und Biotin,
- Pflanzen- und Honigextrakte, wie insbesondere Extrakte aus Eichenrinden, Brennessel, Hamamelis, Hopfen, Kamille, Klettenwurzel, Schachtelhalm, Lindenblüten, Mandel, Aloe Vera, Kokosnuß, Mango, Aprikose, Limone, Weizen, Kiwi, Melone, Orange, Grapefruit, Salbei, Rosmarin, Birke, Wiesenschaumkraut, Quendel, Schafgarbe. Hauhechel, Meristem, Ginseng und Ingwerwurzel,
- Weitere Wirkstoffe wie Ceramide, Allantoin, Pyrrolidoncarbonsäuren, und Bisabolol,
- Lichtschutzmittel,
- Entschäumer wie Silikone,
- Verdickungsmittel wie Agar-Agar, Guar-Gum, Alginate, Xanthan-Gum, Gummi arabicurn, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Alkalisierungsmittel wie beispielsweise Ammoniak, Monoethanolamin, 2-Amino-2-methylpropanol und 2-Amino-2-methyl-propandiol-1,3
- weitere Substanzen zur Einstellung des pH-Wertes,
- Cholesterin,
- Quell- und Penetrationsstoffe wie Glycerin, Propylenglykolmonoethylether, Carbonate, Hydrogencarbonate, Guanidine. Harnstoffe sowie primäre, sekundäre und tertiäre Phosphate,
- Konsistenzgeber wie Zuckerester, Polyolester oder Polyolalkylether,
- Strukturanten wie Maleinsäure, Mono-, Di- und Oligosaccharide,
- Fette und Wachse, wie Walrat, Bienenwachs, Montanwachs und Paraffine,
- Fettsäurealkanolamide,
- Trübungsmittel wie Latex, Styrol/PVP- und Styrol/Acrylamid-Copolymere.
- Perlglanzmittel wie Ethylenglykolmono- und -distearat sowie PEG-3-distearat,
- Komplexbildner wie EDTA, NTA, β-Alanindiessigsäure und Phosphonsäuren,
- Reduktionsmittel wie z. B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.
- Treibmittel wie Propan-Butan-Gemische, N₂O, Dimethylether, CO₂, N₂ und Luft

Bezüglich weiterer Bestandteile sowie Mengenbereiche für die einzelnen Inhaltsstoffe wird auf die dem Fachmann bekannten Handbücher, z. B. K. Schrader, Grundlagen und Rezepturen der Kosmetika, 2. Auflage, Hüthig Buch Verlag, Heidelberg, 1989, verwiesen.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Natriumborat in Frage. Weiterhin ist es möglich, die Oxidation weiterhin mit Hilfe von Enzymen durchzuführen. Dabei können die Enzyme sowohl zur Erzeugung von oxidierenden Perverbindungen eingesetzt werden, als auch zur Verstärkung der Wirkung einer geringen Menge vorhandener Oxidationsmittel. Ein Beispiel für ein enzymatisches Verfahren stellt das Vorgehen dar, die Wirkung geringer Mengen (z. B. 1 % und weniger, bezogen auf das gesamte Mittel) Wasserstoffperoxid durch Peroxidasen zu verstärken.

Die Übergangsmetallkomplexe können erfindungsgemäß sowohl in einer gemeinsamen Zubereitung mit den Farbstoffvorprodukten als auch separat konfektioniert sein.

In einer bevorzugten Ausführungsform der erfindungsgemäßen Lehre werden die separat konfektionierten Übergangsmetallkomplexe in einem geeigneten Lösemittel, beispielsweise in Wasser, Ethanol oder Aceton, gelöst und unmittelbar vor dem Färben der Haare mit der Oxidationsmittelzubereitung verrührt. Diese Zubereitung wird anschließend mit einer Zubereitung, enthaltend die Farbstoffvorprodukte, vermischt. Das dabei entstehende gebrauchsfertige Haarfärbepräparat, auch als Anwendungszubereitung bezeichnet. sollte bevorzugt einen pH-Wert im Bereich von 6 bis 10 aufweisen. Besonders bevorzugt ist ein pH-Wert von 6,5 bis 8. Die Anwendungstemperaturen können in einem Bereich zwischen 15 und 40 °C liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

Durch die erfindungsgemäße Verwendung der Übergangsmetall-Komplexe ist es möglich
- die üblicherweise nur unter alkalischen Bedingungen erhaltenen Färbeleistungen auch im neutralen pH-Bereich zu erzielen oder
- eine schonendere Färbebehandlung dadurch zu erzielen, daß im alkalischen Bereich die angestrebte Färbeleistung bereits mit deutlich, d. h. bis zu 75 %, niedrigeren Konzentrationen an Oxidationsmittel erreich wird. So kann beispielsweise die Wasserstoffperoxidkonzentration in einer Anwendungszubereitung von ca. 3 Gew.-% auf ca. 1,0 Gew.-% gesenkt werden.
- Färbemittel gänzlich ohne chemische Oxidationsmittel zu formulieren.

Ein dritter Gegenstand der Erfindung ist daher ein Verfahren zum Färben keratinhaltiger Fasern, bei dem zunächst eine Zubereitung enthaltend mindestens ein Oxidationsfarbstoffvorprodukt und eine Zubereitung eines Oxidationsmittels zu einer Anwendungszubereitung gemischt werden, diese Anwendungszubereitung anschließend auf die keratinische Faser aufgebracht und schließlich nach einer Einwirkzeit wieder ausgespült wird, dadurch gekennzeichnet, daß die Anwendungszubereitung eine Verbindung der Formel (I) enthält und einen pH-Wert von 6,5 bis 8 aufweist.

Ein vierter Gegenstand der Erfindung ist ein Verfahren zum Färben keratinhaltiger Fasern, bei dem zunächst eine Zubereitung enthaltend mindestens ein Oxidationsfarbstoffvorprodukt und eine Zubereitung eines Oxidationsmittels zu einer Anwendungszubereitung gemischt werden, diese Anwendungszubereitung anschließend auf die keratinische Faser aufgebracht und schließlich nach einer Einwirkzeit wieder ausgespült wird, dadurch gekennzeichnet, daß die Anwendungszubereitung eine Verbindung der Formel (I) enthält und einen pH-Wert von 8,5 bis 10 aufweist.

Ein fünfter Gegenstand der Erfindung schließlich ist ein Verfahren zum Färben keratinhaltiger Fasern, bei dem gewünschtenfalls zunächst eine Zubereitung enthaltend mindestens ein Oxidationsfarbstoffvorprodukt und mindestens eine weitere Zubereitung zu einer Anwendungszubereitung gemischt werden, diese Anwendungszubereitung anschließend auf die keratinische Faser aufgebracht und schließlich nach einer Einwirkzeit wieder ausgespült wird, dadurch gekennzeichnet, daß die Anwendungszubereitung eine Verbindung der Formel (I) enthält und frei von chemischen Oxidationsmitteln ist.

Insbesondere bei schwer färbbarem Haar kann die Zubereitung mit den Oxidationsfarbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert.

Die erfindungsgemäße Lehre umfaßt weiterhin die Möglichkeit, einen Vorläufer der Komplex-Verbindung gemäß Formel (I) in Form eines entsprechenden Komplexes einzusetzen, dem der Ligand A fehlt und in dem das Übergangsmetall eine niedrigere Oxidationsstufe, zum Beispiel +2, aufweist, aus dem sich unter Einwirkung der Persauerstoffverbindung der Komplex gemäß Formel (I) bildet. Weiterhin ist es im Rahmen der erfindungsgemäßen Lehre möglich, die Komplexe gemäß Formel (I) an Trägerstoffen fixiert einzusetzen. Geeignete Trägerstoffe sind beispielsweise Zeolithe, mesoporöse Silikate, Schichtsilikate, Aluminiumphosphate und Aluminiumhydroxide.

### Beispiele

Es wurde eine Färbecreme folgender Zusammensetzung hergestellt (alle Angaben sind, soweit nicht anders vermerkt, in g):

| Komponente | Menge |
|---|---|
| Hydrenol®D¹ | 8,50 |
| Lorol® techn.² | 2,00 |
| Texapon®N 25³ | 20,00 |
| Dehyton®K⁴ | 12,50 |
| Eumulgin®B 2⁵ | 0,75 |
| p-Amino-o-kresol | 0,51 |
| 4-Amino-2-aminomethylphenol-hydrochlorid | 0,79 |
| Turpinal®SL⁶ | 0,16 |
| Ascorbinsäure | 0,53 |
| Ammoniak (25 %ig) | 2 ml |
| Ammoniumdihydrogenphosphat | 0,80 |
| Natriumsulfit | 0,53 g |
| Propylenglykol | 1,52 |
| Wasser | ad 133 |

| | |
|---|---|
| ¹ C16-18-Fettalkohol (HENKEL) | |
| ² C12-18-Fettalkohol (INCI-Bezeichnung: Coconut alcohole) (HENKEL) | |
| ³ Natriumlaurylethersulfat (ca. 28 % Aktivsubstanz in Wasser; INCI-Bezeichnung: Sodium Laureth Sulfate) (HENKEL) | |
| ⁴ N,N-Dimethyl-N(C8-18-kokosamidopropyl)ammoniumacetobetain (ca. 30 % Aktivsubstanz in Wasser ; INCO-Bezeichnung: Cocamidopropyl Betaine) (HENKEL) | |
| ⁵ Cetylstearylalkohol + 20 EO (INCI-Bezeichnung: Ceteareth-20) (HENKEL) | |
| ⁶ 1-Hydroxyethan-1,1-diphosphonsäure (ca. 60 % Aktivsubstanz in Wasser, INCI-Bezeichnung: Etidronic Acid) (HENKEL) | |

Die Creme hatte einen pH-Wert von 8,6.

Als Entwickler wurden 10 ml einer 3 Gew.-%igen polyacrylathaltigen, wäßrigen Lösung von Wasserstoffperoxid verwendet. Der Übergangsmetall-Komplex wurde in einigen Tropfen Wasser vorgelöst und zu der Entwicklerlösung gegeben. Sodann wurden Entwicklerlösung und Färbecreme vereinigt. Der pH-Wert dieser Anwendungszubereitung betrug 7,0.

Sodann wurde die Anwendungszubereitung auf eine Haarsträhne der Sorte "Kerling Naturweiß" (1,5 g Anwendungszubereitung pro g Haar), gegeben, dort 30 Minuten belassen und dann mit Wasser ausgespült.

Es wurden folgende Übergangsmetall-Komplexe verwendet:
K1: {N,N'-Bis[(2-hydroxyphenyl)methylen]-1,2-diaminocyclohexan}mangan(III)-chlorid (Formel (I) mit UM = Mn, R = 1,2-Cyclohexylen, Y¹ = Y² = R¹ = R² = X = Z¹ = Z² = H, A = Chlorid),
K2: {N,N'-Bis[(2-hydroxy-5-nitrophenyl)-methylen]-1,2-diaminocyclohexan}-mangan-(III)-acetat (Formel (I) mit UM = Mn, R = 1,2-Cyclohexylen, Y¹ = Y² = NO₂, R¹ = R² = X = Z¹ = Z² = H, A = Acetat),

Die Ergebnisse der Ausfärbungen sind in der folgenden Tabelle zusammengestellt:

| Komplex | Menge Übergangsmetall(III) in der Anwendungszubereitung | pH-Wert | Ausfärbung |
|---|---|---|---|
| K1 | 110 ppm | 7,0 | intensiv rot |
| K1 | 220 ppm | 7,0 | intensiv rot |
| K2 | 110 ppm | 7,0 | intensiv rot |
| K2 | 220 ppm | 7,0 | intensiv rot |
| - | - | 7,0 - 7,5 | keine Färbung |
| - | - | 9,0 - 9,5 | intensiv rot |

## Patentansprüche

1. Verwendung von Übergangsmetall(III)-Komplexen der Formel (I), 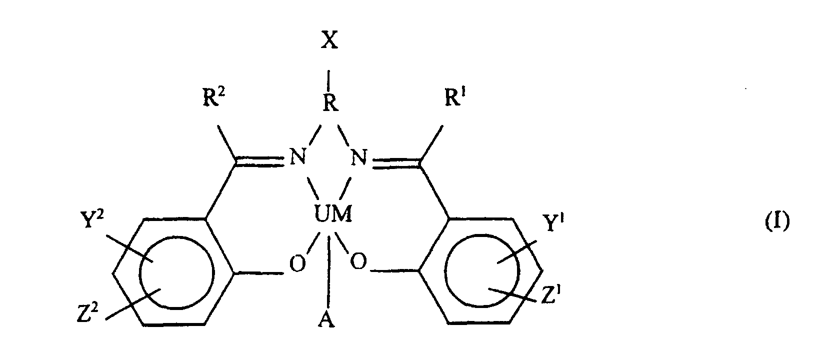 in der
UM für Mangan, Eisen, Kobalt, Ruthenium, Molybdän oder Vanadium steht,
R für einen Alkylen-, Alkenylen-, Phenylen- oder Cycloalkylenrest steht, welcher zusätzlich zum Substituenten X gegebenfalls alkyl- und/oder arylsubstituiert sein kann, mit insgesamt 1 bis 12 C-Atomen, wobei innerhalb R der kürzeste Abstand zwischen den mit UM komplexierenden N-Atomen 1 bis 5 C-Atome beträgt,
X für -H, -OR³, -NO₂, -F, -Cl, -Br oder -J steht,
R¹, R² und R³ unabhängig voneinander für Wasserstoff oder einen Alkylrest mit 1 bis 4 C-Atomen stehen,
Y¹ und Y² unabhängig voneinander für Wasserstoff oder einen elektronenverschiebenden Substituenten stehen,
Z¹ und Z² unabhängig voneinander für Wasserstoff, -CO₂M, -SO₃M, -NO₂, -CH₃, -C₂H₅, -C₃H₇, -CH₂OH oder -CHO stehen,
M für Wasserstoff oder ein Alkalimetall wie Lithium, Natrium oder Kalium steht und
A für einen ladungsausgleichenden Anionliganden steht,
zum Färben keratinhaltiger Fasern.

2. Verwendung nach Anspruch 1, **dadurch gekennzeichnet, daß** das Übergangsmetall UM in der Verbindung nach Formel (I) Mangan ist.

3. Verwendung nach einem der Ansprüche 1 oder 2. **dadurch gekennzeichnet, daß** in Formel (I) R eine Methylengruppe, 1,2-Ethylengruppe, 1,3-Propylengruppe, in Position 2 hydroxy- oder nitrosubstituierte 1,3-Propylengruppe, 1,2-Cylcloalkylengruppe mit 4 bis 6 C-Atomen, insbesondere eine 1,2-Cyclohexylengruppe, oder eine o-Phenylengruppe ist.

4. Verwendung nach einem der Ansprüche 1 bis 3, **dadurch gekennzeichnet, daß** Y¹ und Y² in Formel (I) unter Wasserstoff, der Hydroxygruppe, Alkoxygruppen mit 1 bis 4 C-Atomen, Aryloxgruppen, der Nitrogruppe, den Halogenen, der Aminogruppe, welche auch mono- oder dialkyliert oder -aryliert sein kann, den linearen oder verzweigtkettigen Alkylgruppen mit 1 bis 4 C-Atomen, Cycloalkylgruppen mit 3 bis 6 C-Atomen, linearen oder verzweigtkettigen Alkenylgruppen mit 2 bis 5 C-Atomen, und Arylgruppen, welche ihrerseits die vorgenannten Substituenten tragen können, ausgewählt werden.

5. Verwendung nach einem der Ansprüche 1 bis 4, **dadurch gekennzeichnet, daß** Y¹ und Y² in Formel (I) unter den Alkenylgruppen, welche 1 oder 2 C-C-Doppelbindungen enthalten, wobei mindestens eine Doppelbindung in Konjugation zum Benzolring steht, ausgewählt werden.

6. Verwendung nach Anspruch 5, **dadurch gekennzeichnet, daß** die Alkenylgruppe die Allyl- oder Vinylgruppe ist.

7. Verwendung nach einem der Ansprüche 1 bis 6, **dadurch gekennzeichnet, daß** in den Verbindungen der Formel (I) die Substituenten Y¹ und Y² in 5-Stellung stehen und/oder identisch sind.

8. Verwendung nach einem der Ansprüche 1 bis 7, **dadurch gekennzeichnet, daß** der ladungsausgleichende Anionligand A in den Verbindungen der Formel (I) ein- oder mehrwertig ist, wobei es im letzteren Fall entsprechend mehrere Übergangsmetallatome mit den Salen-Liganden neutralisiert und insbesondere ein Halogenid, ein Hydroxid, Hexafluorophosphat, Perchlorat oder das Anion einer Carbonsäure ist.

9. Verwendung nach einem der Ansprüche 1 bis 8, **dadurch gekennzeichnet, daß** R¹, R² und R³ unabhängig voneinander aus Methyl-, Ethyl-, n-Propyl-, iso-Propyl-, n-Butyl-, sec-Butyl-, iso-Butyl- und tert-Butyl-Gruppen ausgewählt werden.

10. Mittel zum Färben keratinischer Fasern, insbesondere menschlicher Haare, enthaltend mindestens ein Oxidationsfarbstoffvorprodukt, **dadurch gekennzeichnet, daß** es 0,001 bis 1,0 Gew.-% einer Verbindung der Formel (I) enthält.

11. Mittel nach Anspruch 10, **dadurch gekennzeichnet, daß** es mindestens ein Oxidationsfarbstoffvorprodukt vom Typ der Entwickler enthält.

12. Mittel nach Anspruch 10 oder 11, **dadurch gekennzeichnet, daß** es mindestens ein Indol- oder Indolinderivat enthält, das mindestens einen Hydroxysubstituenten trägt.

13. Verfahren zum Färben keratinhaltiger Fasern, bei dem zunächst eine Zubereitung enthaltend mindestens ein Oxidationsfarbstoffvorprodukt und eine Zubereitung eines Oxidationsmittels zu einer Anwendungszubereitung gemischt werden, diese Anwendungszubereitung anschließend auf die keratinische Faser aufgebracht und schließlich nach einer Einwirkzeit wieder ausgespült wird, **dadurch gekennzeichnet, daß** die Anwendungszubereitung eine Verbindung der Formel (I) enthält und einen pH-Wert von 6,5 bis 8 aufweist.

14. Verfahren zum Färben keratinhaltiger Fasern, bei dem zunächst eine Zubereitung enthaltend mindestens ein Oxidationsfarbstoffvorprodukt und eine Zubereitung eines Oxidationsmittels zu einer Anwendungszubereitung gemischt werden, diese Anwendungszubereitung anschließend auf die keratinische Faser aufgebracht und schließlich nach einer Einwirkzeit wieder ausgespült wird, **dadurch gekennzeichnet, daß** die Anwendungszubereitung eine Verbindung der Formel (I) enthält und einen pH-Wert von 8,5 bis 10 aufweist.

15. Verfahren zum Färben keratinhaltiger Fasern, bei dem gewünschtenfalls zunächst eine Zubereitung enthaltend mindestens ein Oxidationsfarbstoffvorprodukt und mindestens eine weitere Zubereitung zu einer Anwendungszubereitung gemischt werden, diese Anwendungszubereitung anschließend auf die keratinische Faser aufgebracht und schließlich nach einer Einwirkzeit wieder ausgespült wird, **dadurch gekennzeichnet, daß** die Anwendungszubereitung eine Verbindung der Formel (I) enthält und frei von chemischen Oxidationsmitteln ist.

## Claims

1. The use of transition metal(III) complexes corresponding to formula (I): 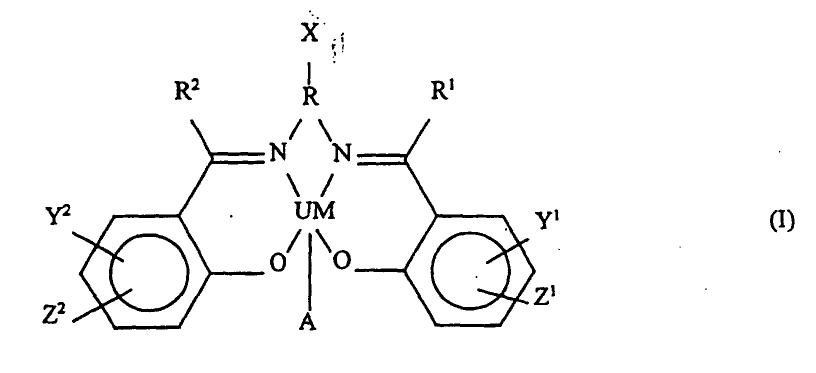 in which
UM stands for manganese, iron, cobalt, ruthenium, molybdenum or vanadium,
R is an alkylene, alkenylene, phenylene or cycloalkylene group which, in addition to the substituent X, may optionally be alkyl- and/or aryl-substituted and which has a total of 1 to 12 carbon atoms, the shortest distance in R between the N atoms complexing with UM being 1 to 5 carbon atoms,
X stands for -H, -OR³, -NO₂, -F, -Cl, -Br or -I,
R¹, R² and R³ independently of one another represent hydrogen or an alkyl group containing 1 to 4 carbon atoms,
Y¹ and Y² independently of one another represent hydrogen or an electron-shifting substituent,
Z¹ and Z² independently of one another represent hydrogen, -CO₂M, -SO₃M, -NO₂, -CH₃, -C₂H₅, -C₃H₇, -CH₂OH or -CHO,
M stands for hydrogen or an alkali metal, such as lithium, sodium or potassium, and
A is a charge-compensating anion ligand,
for coloring keratin-containing fibers.

2. The use claimed in claim 1, **characterized in that** the transition metal UM in the compound of formula (I) is manganese.

3. The use claimed in claim 1 or 2, **characterized in that** R in formula (I) is a methylene group, a 1,2-ethylene group, a 1,3-propylene group, a 2-hydroxy- or 2-nitro-substituted 1,3-propylene group, a 1,2-cycloalkylene group containing 4 to 6 carbon atoms, more particularly a 1,2-cyclohexylene group, or an o-phenylene group.

4. The use claimed in any of claims 1 to 3, **characterized in that** Y¹ and Y² in formula (I) are selected from hydrogen, the hydroxy group, alkoxy groups containing 1 to 4 carbon atoms, aryloxy groups, the nitro group, the halogens, the amino group which may be mono- or dialkylated or mono- or diarylated, linear or branched alkyl groups containing 1 to 4 carbon atoms, cycloalkyl groups containing 3 to 6 carbon atoms, linear or branched alkenyl groups containing 2 to 5 carbon atoms and aryl groups which, in turn, may carry the above-mentioned substituents.

5. The use claimed in any of claims 1 to 4, **characterized in that** Y¹ and Y² in formula (I) are selected from alkenyl groups containing 1 or 2 C-C double bonds, at least one double bond being conjugated to the benzene ring.

6. The use claimed in claim 5, **characterized in that** the alkenyl group is the allyl or vinyl group.

7. The use claimed in any of claims 1 to 6, **characterized in that**, in the compounds of formula (I), the substituents Y¹ and Y² are in the 5 position and/or are identical.

8. The use claimed in any of claims 1 to 7, **characterized in that** the charge-compensating anion ligand A in the compounds corresponding to formula (I) is mono- or polyvalent; in the latter case, it correspondingly neutralizes several transition metal atoms with the salen ligands and, in particular, is a halide, a hydroxide, hexafluorophosphate, perchlorate or the anion of a carboxylic acid.

9. The use claimed in any of claims 1 to 8, **characterized in that** R¹, R² and R³ independently of one another are selected from methyl, ethyl, n-propyl, isopropyl, n-butyl, sec.butyl, isobutyl and tert.butyl groups.

10. A composition for coloring keratin fibers, more particularly human hair, containing at least one oxidation dye precursor, **characterized in that** it contains 0.001 to 1.0% by weight of a compound corresponding to formula (I).

11. A composition as claimed in claim 10, **characterized in that** it contains at least one oxidation dye precursor of the primary intermediate type.

12. A composition as claimed in claim 10 or 11, **characterized in that** it contains at least one indole or indoline derivative bearing at least one hydroxy substituent.

13. A process for coloring keratin-containing fibers in which a preparation containing at least one oxidation dye precursor and a preparation of an oxidizing agent are first mixed to form a ready-to-use preparation which is then applied to the keratin fibers and, finally, is rinsed out after a certain contact time, **characterized in that** the ready-to-use preparation contains a compound corresponding to formula (I) and has a pH of 6.5 to 8.

14. A process for coloring keratin-containing fibers in which a preparation containing at least one oxidation dye precursor and a preparation of an oxidizing agent are first mixed to form a ready-to-use preparation which is then applied to the keratin fibers and, finally, is rinsed out after a certain time, **characterized in that** the ready-to-use preparation contains a compound corresponding to formula (I) and has a pH of 8.5 to 10.

15. A process for coloring keratin-containing fibers in which, if desired, a preparation containing at least one oxidation dye precursor and at least one other preparation are first mixed to form a ready-to-use preparation which is then applied to the keratin fibers and, finally, is rinsed out again after a certain contact time, **characterized in that** the ready-to-use preparation contains a compound corresponding to formula (I) and is free from chemical oxidizing agents.

## Revendications

1. Utilisation de complexes de métaux de transition (III) de formule (I), 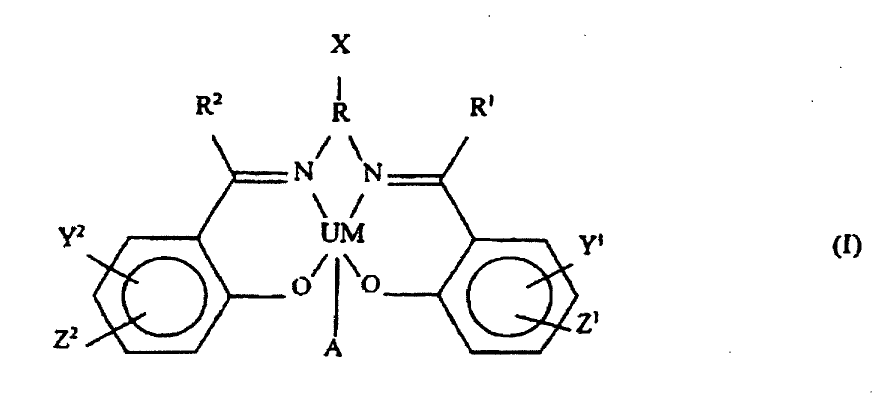 dans laquelle
UM représente le manganèse, le fer, le cobalt, le ruthénium, le molybdène ou le vanadium,
R représente un groupe alkylène, alcénylène, phénylène ou cycloalkylène qui, en plus de substituants X, peut porter éventuellement des substituants alkyle et/ou aryle, comprenant au total 1 à 12 atomes de C, la plus courte distance à l'intérieur de R qui sépare les atomes de N à complexer avec UM étant de 1 à 5 atomes de C,
X représente -H, -OR³, -NO₂, -F, -Cl, -Br ou -I,
R¹, R² et R³ représentent indépendamment un atome d'hydrogène ou un groupe alkyle comprenant de 1 à 4 atomes de C,
Y¹ et Y² représentent indépendamment un atome d'hydrogène ou un substituant déplaçant les électrons,
Z¹ et Z² représentent indépendamment un atome d'hydrogène, -CO₂M, -SO₃M, -NO₂, -CH₃, -C₂H₅, -C₃H₇, -CH₂OH ou -CHO,
M représente un atome d'hydrogène ou un métal alcalin tel que le lithium, le sodium ou le potassium et
A représente un ligand d'anion d'équilibrage des charges,
pour la teinture de fibres kératiniques.

2. Utilisation selon la revendication 1, **caractérisée en ce que** dans le composé de formule (I), le métal de transition UM est le manganèse.

3. Utilisation selon l'une des revendications 1 ou 2, **caractérisée en ce que** dans la formule (I), R représente un groupe méthylène, 1,2-éthylène, 1,3-propylène, 1,3-propylène substitué en position 2 par un groupe hydroxy ou nitro, 1,2-cylcloalkylène comprenant 4 à 6 atomes de C, en particulier un groupe 1,2-cylclohexylène, ou un groupe o-phénylène.

4. Utilisation selon l'une quelconque des revendications 1 à 3, **caractérisée en ce que** dans la formule (I), Y¹ et Y² peuvent être choisis parmi un atome d'hydrogène, un groupe hydroxy, un groupe alcoxy comprenant 1 à 4 atomes de C, un groupe aryloxy, nitro, un atome d'halogène, un groupe amino qui peut être également mono- ou dialkylé ou mono- ou diarylé, des groupes alkyle linéaires ou ramifiés comprenant 1 à 4 atomes de C, des groupes cycloalkyle comprenant de 3 à 6 atomes de C, des groupes alcényle linéaires ou ramifiés comprenant de 2 à 5 atomes de C et des groupes aryle, qui à leur tour peuvent porter les substituants mentionnés ci-dessus.

5. Utilisation selon l'une quelconque des revendications 1 à 4, **caractérisée en ce que** dans la formule (I), Y¹ et Y² peuvent être choisis parmi des groupes alcényle qui comprennent 1 ou 2 double liaisons C-C, au moins une des doubles liaisons étant conjuguée avec le noyau benzénique.

6. Utilisation selon la revendication 5, **caractérisée en ce que** le groupe alcényle est un groupe allyle ou vinyle.

7. Utilisation selon l'une quelconque des revendications 1 à 6, **caractérisée en ce que** dans les composés de formule (I), les substituants Y¹ et Y² sont en position 5 et/ou sont identiques.

8. Utilisation selon l'une quelconque des revendications 1 à 7, **caractérisée en ce que** dans les composés de formule (I), le ligand d'anion d'équilibrage des charges A est monovalent ou à valences multiples, et dans ce dernier cas, il peut neutraliser plusieurs atomes de métaux de transition avec les ligands salins, et en particulier, il peut être un halogénure, un hydroxyde, un hexafluorophosphate, un perchlorate ou l'anion d'un acide carboxylique.

9. Utilisation selon l'une quelconque des revendications 1 à 8, **caractérisée en ce que** R¹, R² et R³ sont choisis indépendamment parmi les groupes méthyle, éthyle, n-propyle, iso-propyle, n-butyle, sec-butyle, iso-butyle et tert-butyle.

10. Agent de teinture de fibres kératiniques, en particulier de cheveux humains, contenant au moins un précurseur de colorant oxydant, **caractérisé en ce qu'**il contient de 0,001 à 1,0 % en poids d'un composé de formule (I).

11. Agent selon la revendication 10, **caractérisé en ce qu'**il contient au moins un précurseur de colorant oxydant de type développeur.

12. Agent selon la revendication 10 ou 11, **caractérisé en ce qu'**il contient au moins un dérivé d'indole ou d'indoline qui porte au moins un substituant hydroxy.

13. Procédé de teinture de fibres kératiniques, dans lequel on mélange d'abord une préparation contenant au moins un précurseur de colorant oxydant et une préparation d'un agent oxydant pour obtenir une préparation d'application, ensuite on applique cette préparation d'application sur des fibres kératiniques et enfin, après un temps de pose, on rince, **caractérisé en ce que** la préparation d'application contient un composé de formule (I) et présente un pH de 6,5 à 8.

14. Procédé de teinture de fibres kératiniques, dans lequel on mélange d'abord une préparation contenant au moins un précurseur de colorant oxydant et une préparation d'un agent oxydant pour obtenir une préparation d'application, ensuite on applique cette préparation d'application sur des fibres kératiniques et enfin, après un temps de pose, on rince, **caractérisé en ce que** la préparation d'application contient un composé de formule (I) et présente un pH de 8,5 à 10.

15. Procédé de teinture de fibres kératiniques, dans lequel, selon le besoin, on mélange d'abord une préparation contenant au moins un précurseur de colorant oxydant et au moins une autre préparation pour obtenir une préparation d'application, ensuite on applique cette préparation d'application sur des fibres kératiniques et enfin, après un temps de pose, on rince, **caractérisé en ce que** la préparation d'application contient un composé de formule (I) et est exempte d'agent oxydant chimique.
